(19) Europäisches Patentamt
European Patent Office
Office européen des brevets

(11) EP 3 172 375 B1

(12) **FASCICULE DE BREVET EUROPEEN**

(45) Date de publication et mention de la délivrance du brevet:
**13.03.2024 Bulletin 2024/11**

(21) Numéro de dépôt: **15753392.8**

(22) Date de dépôt: **20.07.2015**

(51) Classification Internationale des Brevets (IPC):
***A61K 8/02*** *(2006.01)* ***A61K 8/11*** *(2006.01)*
***A61Q 19/00*** *(2006.01)* ***B01J 13/08*** *(2006.01)*
***C11D 17/00*** *(2006.01)* ***A01N 25/28*** *(2006.01)*
***D06M 23/12*** *(2006.01)* ***B01J 13/18*** *(2006.01)*
***B01J 13/22*** *(2006.01)* ***C11D 17/04*** *(2006.01)*

(52) Classification Coopérative des Brevets (CPC):
(C-Sets disponibles)
**A61K 8/11; A01N 25/28; A61K 8/0208; A61Q 19/00; B01J 13/08; B01J 13/18; B01J 13/22; C11D 17/0039; C11D 17/049; D06M 23/12;**
A61K 2800/412 (Cont.)

(86) Numéro de dépôt international:
**PCT/FR2015/051997**

(87) Numéro de publication internationale:
**WO 2016/012711 (28.01.2016 Gazette 2016/04)**

(54) **SUPPORT FIBREUX COMPORTANT DES PARTICULES CONTENANT UN AGENT ACTIF PARTIELLEMENT SOLUBLE DANS L'EAU, PARTICULES ET MÉTHODES DE FABRICATION DES PARTICULES**

FASERTRÄGER MIT PARTIKELN MIT EINEM TEILWEISE WASSERLÖSLICHEN WIRKSTOFF, PARTIKEL UND VERFAHREN ZUR HERSTELLUNG BESAGTER PARTIKEL

FIBROUS SUPPORT COMPRISING PARTICLES CONTAINING A PARTIALLY WATER-SOLUBLE ACTIVE AGENT, PARTICLES, AND METHODS FOR PRODUCING SAID PARTICLES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **21.07.2014 FR 1457030**

(43) Date de publication de la demande:
**31.05.2017 Bulletin 2017/22**

(73) Titulaire: **Satisloh AG**
**6340 Baar (CH)**

(72) Inventeurs:
- **CADET, Mamonjy**
**94220 Charenton-le-Pont (FR)**
- **MERIDIANO, Camille**
**94220 Charenton-le-Pont (FR)**
- **HUILIER, Hervé**
**70400 Errevet (FR)**
- **CALLET, Adeline**
**25400 Exincourt (FR)**

(74) Mandataire: **Jacobacci Coralis Harle**
**32, rue de l'Arcade**
**75008 Paris (FR)**

(56) Documents cités:
**EP-A1- 1 533 415** **EP-A2- 0 407 257**
**FR-A1- 2 995 222** **GB-A- 1 242 689**
**JP-A- H02 300 387** **JP-A- 2004 360 157**
**US-A1- 2002 055 560** **US-A1- 2011 200 654**
**US-B2- 6 951 836**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

(52) Classification Coopérative des Brevets (CPC):
(Cont.)

C-Sets
**A01N 25/28, A01N 37/46**

## Description

La présente divulgation concerne de manière générale des supports en fibres naturelles et/ou synthétiques, de préférence souples, dans lesquels sont retenus des particules comprenant en leur sein au moins un agent actif partiellement soluble dans l'eau, les particules libérant, au moins en partie, le (ou les) agent(s) actif(s) sous l'effet d'une contrainte externe, ainsi que le procédé de fabrication de tels supports.

La divulgation concerne également des particules comprenant au moins un agent actif partiellement soluble dans l'eau ainsi que leurs procédés de fabrication.

On connait des textiles fonctionnels capables de délivrer sur une surface un agent actif retenu par le textile. L'agent actif peut être de nature variée, par exemple un agent détergent, antibuée, bactéricide, biocide, un principe actif thérapeutique dermatologique ou cosmétique (hydratant, émollient, nettoyant, relipidant, lipolytique, exfoliant, tenseur, pigmentant ou dépigmentant, antiseptique, désinfectant, cicatrisant), parfum et odorisant.

La surface sur laquelle on délivre l'agent actif peut être de toute nature, par exemple la peau, un verre minéral ou organique, un métal ou un alliage, etc...

Un domaine plus particulier de l'invention est celui des textiles fonctionnels utilisés en optique, notamment en optique ophtalmique, pour nettoyer les surfaces de l'article d'optique ou lui conférer < ou rétablir > des propriétés spécifiques, en particulier pour conférer ou rétablir à ces surfaces des propriétés antibuée.

Les tissus antibuée connus, également dénommés lingettes antibuée, sont obtenus par imprégnation de l'agent actif liquide dans les tissus.

Ce type de lingettes antibuée est décrit entre autres dans le brevet FR 2 868 684 et la demande de brevet JP 2009/195648 et la demande de brevet WO2013013929. Dans ce cas, l'agent actif n'est pas protégé et peut donc être dégradé du fait d'interactions physiques ou chimiques avec le milieu extérieur (érosion, rayonnement, humidité, etc.). Un lavage de ces lingettes éliminerait l'agent antibuée présent (en particulier si l'agent est partiellement hydrosoluble) et ferait perdre la fonction de recharge antibuée à la lingette. La durabilité de ces lingettes est donc limitée par leur non-résistance au lavage.

Il a été proposé d'immobiliser l'agent antibuée sur le textile au sein de microcapsules. De cette manière, l'agent antibuée est alors libéré vers la surface à traiter par rupture de la membrane de la microcapsule ou par érosion de la microsphère lors de l'exercice d'une pression du textile sur le verre. L'agent antibuée est donc délivré de manière contrôlée lors de l'utilisation du textile et n'est pas lessivé par un éventuel lavage, ou un contact abondant avec de l'eau.

Ainsi, le brevet japonais JP 02300387 décrit un textile microfibre dans lequel sont immobilisés des microcapsules contenant un agent antibuée. L'agent d'encapsulation est du polyuréthane ou une résine urée-formaldéhyde. L'agent antibuée est un tensio-actif à base de fluor. Il n'y a pas d'indication que l'agent tensioactif soit partiellement soluble dans l'eau. Le brevet JP 2005 296920 décrit un textile revêtu d'agent antibuée ayant une durabilité améliorée vis-à-vis de l'usure et du lavage. Comme agent antibuée de nombreux tensio-actifs sont cités. Le textile est un textile acrylique et les capsules sont en silice poreuse, gélatine, résines urée-mélamine, polyurée.

Le brevet EP 2080552 décrit un procédé d'encapsulation dans une membrane de polysiloxane d'un composé soit hydrophile soit hydrophobe. Il n'est pas fait mention d'agents actifs ayant une solubilité partielle dans l'eau.

La demande de brevet WO 2012 038666 décrit également la fabrication de microcapsules de polysiloxane qui peuvent être fonctionnalisées pour se greffer sur des fibres textiles ayant des fonctions hydroxyles. Là encore, l'agent actif est soit hydrophile soit hydrophobe. Il n'est jamais fait mention d'un agent actif partiellement hydrosoluble.

Le brevet FR 2 995 222 décrit un procédé de microencapsulation par « congélation » de goutte. L'agent actif est soit liposoluble, soit lipodispersable, soit non hydrosoluble. Un agent actif partiellement hydrosoluble n'est jamais envisagé.

Le brevet EP 1533 415 décrit des microcapsules de mélamine-formaldéhyde contenant un principe actif liposoluble, lipodispersable ou non hydrosoluble. Les brevets EP0407257A, US2002/055560 A, GB1242689A, FR2995222A, JPH02300387A, EP1533415A, JP2004360157A, US6951836B et US2011/200654A font aussi partie de l'état de la technique concernant la production de particules contenant des principes actifs.

Les technologies d'encapsulation connues et classiquement utilisées dans le textile ne permettent pas d'encapsuler des agents actifs partiellement solubles dans l'eau comme les tensioactifs, notamment fluorés, comportant une chaine hydrophile (par exemple polyéthylène) ou comme l'éthyl-3 [acétyl (butyl) amino] propanoate (IR 3535 biocide, aussi utilisé comme répulsif à insecte), ou encore comme la caféine.

De manière plus générale, les technologies d'encapsulation classiques passent par la préparation d'émulsions huile-dans-l'eau ou eau-dans-l'huile, qui nécessitent l'ajout de tensioactifs (amphiphiles) pour le contrôle de l'émulsion. L'encapsulation de produits actifs partiellement solubles dans l'eau (pouvant également être amphiphiles) fait que ces molécules se retrouvent alors réparties entre les deux phases de l'émulsion. Or la phase aqueuse (qui est le plus souvent la phase dispersante de l'émulsion) est le lieu de réactions chimiques très sensibles aux conditions du milieu (pH, force ionique, concentration) conduisant à la formation des membranes d'encapsulation. La présence de quantités mesurables de l'agent actif dans cette phase est donc préjudiciable à la formation des capsules.

L'intérêt de l'encapsulation de l'agent actif (par exemple un agent antibuée) est de le protéger vis-à-vis de l'environnement (rayonnement, humidité, réactivité chimique) et également de contrôler son relargage sur la surface par la pression ou par frottement ou par simple contact (par exemple, lors de l'essuyage de verres de lunette, application du textile sur la peau ou un substrat).

En outre, il est important que les agents actifs soient principalement situés au coeur des particules et non à leurs périphéries pour un usage prolongé des lingettes.

Un premier objet de la présente divulgation est de fournir un support constitué de fibres pour principes actifs alternatif à ceux existant dans l'art antérieur.

Un autre objet de la présente divulgation est de fournir un support constitué de fibres sur lequel sont retenues des particules contenant en leur sein au moins un agent actif partiellement soluble dans l'eau et remédiant aux inconvénients de l'art antérieur.

L'invention a également pour objet un procédé de fabrication de particules comprenant en leur sein au moins un agent actif partiellement soluble dans l'eau.

Le but ci-dessus est atteint selon l'invention par la réalisation d'un support en fibres naturelles et/ou synthétiques, de préférence un support souple, dans lesquels sont retenues des particules, de préférence insolubles dans l'eau, comprenant en leur sein au moins un agent actif ayant une solubilité partielle dans l'eau de 0,1 à 60%, de préférence de 0,1 à 30% en poids, lesdites particules libérant, au moins en partie, le (ou les) agent(s) actif(s) sous l'effet d'une contrainte externe.

Définitions et description des éléments de l'invention

Particule : objet de forme quelconque (de préférence sphérique ou quasi sphérique) d'une taille de 100 nm à 200 μm, de préférence de 100 nm à 100 μm et mieux de 100 nm à 50 μm et contenant au moins un agent actif. De préférence, les particules sont insolubles dans l'eau. Le relargage, au moins en partie, de l'agent actif se fait sous l'action d'une contrainte externe, par exemple une contrainte mécanique, thermique ou chimique.

Microcapsule : Particule de forme quelconque (par exemple sphérique ou quasi sphérique) d'une taille de 100 nm à 200 μm définie par une enveloppe ou une membrane par exemple en matériau polymère de type aminoplaste, résine urée-formaldéhyde, résine phénolique, acides gras, esters d'acides gras, cires d'origine végétale, polysiloxanes, organosiloxanes, dérivés de cellulose, gommes d'origines végétale éventuellement modifiées renfermant une phase fluide ou solide dans laquelle est présent l'agent actif, soit à l'état pur, soit solubilisé ou dispersé au sein d'une phase-hôte dans des proportions de préférence de 0,01 à 99,999% en poids. Le ratio en poids contenu/contenant peut varier de 80/20 à 1/999. Le relargage d'au moins une partie de l'agent actif se fait par dégradation de la membrane soit sous contrainte mécanique soit sous contrainte thermique.

Microsphère : Particule de forme quelconque (par exemple sphérique ou quasi sphérique) d'une taille de 100 nm à 200 μm, constituée d'au moins un vecteur appelé matrice par exemple de type acides gras, esters d'acides gras, cires d'origine végétale, polysiloxanes, organosiloxanes, dérivés de cellulose, gommes d'origine végétale éventuellement modifiées et d'au moins un agent actif solubilisé ou dispersé au sein de la matrice dans laquelle l'agent actif est présent soit à l'état pur, soit solubilisé ou dispersé dans une phase-hôte dans des proportions de 0,001 à 99,999 % en poids. Le ratio en poids contenu/matrice peut varier de 60/40 à 1/999. La matrice est non-soluble dans l'eau et est solide à température ambiante (20°C) et de préférence jusqu'à 30 °C voire 35°C. est également soit solide soit non soluble dans l'eau aux températures de fabrication des textiles délivrants.

Le relargage d'au moins une partie de l'agent actif peut s'effectuer par application d'une contrainte thermique (par exemple application d'une température supérieure à la température de fusion ou la température de ramollissement ou la température de transition vitreuse de la matrice, selon la nature de la matrice). Le relargage peut également s'effectuer sous contrainte mécanique, par exemple par frottement, par érosion ou par fluage. La libération de l'agent actif peut aussi avoir lieu sous contrainte chimique, par exemple application du solvant ou modification du pH.

Support

Les supports sont tous les matériaux en fibres, de préférence souples. Ces supports sont généralement les textiles ou papiers non-tissés, les textiles fabriqués par tissage ou par tricotage, les feutres et les ouates de cellulose.

Les fibres peuvent être des fibres naturelles ou synthétiques telles que les fibres de coton, lin, chanvre, jute, soie, laine, cellulose, polyamides, acétate, viscose, modal, acryliques, polyesters et les chlorofibres.

Les supports préférés selon l'invention pour des applications ophtalmiques et/ou antibuée sont les tissus en microfibres tissés ou tricotés, de préférence tricotés.

Ainsi qu'on le sait, un matériau tissé est obtenu par entrecroisement de façon perpendiculaire de deux ensembles de fils dans le sens longitudinal (chaîne) et dans le sens transversal (trame), alors qu'un matériau non tissé est une feuille manufacturée constituée de voiles ou de nappes de fibres orientées ou non, liées par friction, cohésion et/ou

adhésion.

Un tissu tricoté est obtenu par enroulage en boucle d'un ou plusieurs fils pour former des mailles entrelacées les unes dans les autres.

Selon l'invention, pour des applications ophtalmiques et/ou antibuée, on utilise préférentiellement un tissu tricoté avec un nombre de mailles/$cm^2$ d'au moins 300, de préférence au moins 400, mieux au moins 500, mieux encore supérieur à 700. La gamme optimale pour le nombre de mailles/$cm^2$ est supérieure à 800 et mieux encore supérieure à 900 mailles/$cm^2$. En tant que de besoin, l'homme du métier se référera à la norme NF EN 14971, relative à cette caractéristique de l'invention.

Le tissu utilisé dans l'invention comprend de préférence au moins 80% en masse de microfibres, mieux au moins 90% en masse de microfibres, de préférence au moins 95% en masse, mieux 100 % en masse de microfibres. Par microfibres, on entend des fibres textiles dont la masse linéique est inférieure à 1,3 décitex (1,3g /10 km). Les microfibres préférées ont une masse linéique inférieure à 1 décitex.

Le tissu en microfibres comprend selon l'invention des microfibres de polymère hydrophile et des microfibres de polymère lipophile,

Les microfibres de polymère hydrophile présentent une affinité pour l'eau, alors que les microfibres de polymère lipophile présentent une affinité pour les huiles.

Le polymère lipophile présente des affinités avec les salissures de type sébum, alors que le polymère hydrophile présente une affinité vis-à-vis de l'humidité présente à la surface du substrat traité par la lingette, de préférence un verre ophtalmique.

Un polymère hydrophile utilisé préférentiellement est un polymère capable d'un taux de reprise en eau supérieur ou égal à 2%, mieux supérieur ou égal à 3%.

Le taux de reprise en eau est le rapport entre la masse conditionnée d'un échantillon (après 24 heures à 20°C et 65% de taux d'humidité ambiante) et la masse anhydre obtenue en étuve à 105°C+/- 2°C (séchage jusqu'à l'obtention d'une masse constante).

La mesure de reprise en eau est connue de l'homme du métier qui pourra se référer, en tant que de besoin à la norme EN ISO6741.

Il est préférable que le taux de reprise en eau du polymère hydrophile soit inférieur à 10%, mieux inférieur à 8% et mieux encore inférieur ou égal à 7%.

Un polymère lipophile utilisé préférentiellement présente un taux de reprise en eau inférieur à 2%, mieux inférieur à 1,5% et mieux encore inférieur à 1%.

Les polymères hydrophiles préférés sont les polyamides 6.6 (taux de reprise en eau (TRE) de 2,5 à 6%), les polyamides 6 (TRE de 5,75%), les celluloses (TRE de 8% à 13%).

Les polymères lipophiles préférés sont les polyesters (TRE de 0,15% à 0,50%) et les polypropylènes (TRE de 0,05% à 0,50%).

Le tissu en microfibres comprend de préférence des microfibres de polyamide et des microfibres de polyester, mieux de 60 à 85 % en masse de microfibres de polyester et de 15 à 40 % en masse de microfibres de polyamide. Un exemple d'un tel tissu est le tissu Cémoi™, composé de 69,5 % en masse de microfibres de polyester et de 30,5 % en masse de microfibres de polyamide. Un tissu composé de 79 % en masse de microfibres de polyester et de 21 % en masse de microfibres de polyamide, fourni par la société Kelnet, est également approprié.

Préférentiellement, le tissu en microfibres comprend majoritairement des microfibres de section triangulaire. Préférentiellement, au moins 80 % en nombre des microfibres de polymère lipophile sont de section triangulaire.

Les microfibres utilisées peuvent être obtenues par éclatement de fibres, préférentiellement de structure dite en "quartiers d'orange", les quartiers d'orange étant constitués préférentiellement de polymère lipophile. Ainsi, selon un mode de réalisation préférentiel, les microfibres sont obtenues à partir de fibres de structure composite de polymère hydrophile et lipophile, par éclatement de ladite structure composite, après tissage ou tricotage.

Pour des applications dermatologiques, on préfère utiliser des textiles de mêmes compositions que celles des microfibres, mais de dimensions supérieures à celles de microfibres.

<u>Maintien des particules sur le support</u>

Les particules sont retenues au sein du support soit par immobilisation soit par fixation.

<u>Immobilisation</u> : les particules sont déposés sur le support sans volonté de fixation par une liaison covalente ni par l'intermédiaire d'un liant entre les particules et le support. Cette immobilisation peut être réalisée par des techniques classiques d'imprégnation au foulard, par rouleau lécheur, par pulvérisation, par trempage, etc.

<u>Fixation</u> : les particules sont déposées sur le support de manière classique et fixés au support par un traitement adéquat (réticulation thermique, irradiation lumineuse ou photonique) d'un liant ou d'un agent de pontage pour former des liaisons covalentes entre le support et particules. Cette fixation améliore la résistance au lessivage des supports selon l'invention.

Agent actif : L'agent actif est un composé ou mélange de composés qui confère une ou plusieurs propriétés données à un substrat lorsqu'il est appliqué sur ce substrat.

Selon la présente invention, l'agent actif présente une solubilité partielle dans l'eau allant de 0,1 à 60% en poids, de préférence de 0,1 à 30% en poids, de préférence encore de 0,2 à 60% mieux de 0,2 à 40%, encore mieux de 0,2 à 30% en poids à une température T choisie dans la gamme allant de 20°C à 95°C, à une pression d'une atmosphère.

Une autre gamme préférentielle de solubilité dans l'eau est de 0,5 à 60%, de préférence de 1 à 60%, mieux de 2 à 60%, et encore mieux de 2 à 30% en poids à une température T choisie dans la gamme allant de 20°C à 95°C, à unepression d'une atmosphère.

Préférentiellement, la solubilité est mesurée à une température T de 20°C.

Pour les composés solides, la solubilité est définie comme le pourcentage en poids du composé dans une solution à l'équilibre avec la phase solide à la température T ci-dessus et une pression d'une atmosphère. Pour les composés liquides (ou solides à l'état pur) dont les mélanges aqueux se séparent en deux ou plusieurs phases, la solubilité prise en compte est la le pourcentage en poids du composé spécifié dans la phase liquide riche en eau à l'équilibre à la température de travail (température à laquelle sont fabriquées lesdites particules (selon le cas entre 20 et 95°C) et une pression d'une atmosphère. En particulier, l'agent actif peut être un composé amphiphile, notamment une molécule fluorée amphiphile non ionique.

L'agent actif peut être un composé ionique ou non ionique, organique ou organométallique, tensioactif ou non-tensioactif.

L'agent actif peut avoir des propriétés applicatives variées telles que : antibuée, bactéricides, biocides, détergentes, répulsives à insectes, thérapeutiques notamment dermatologiques ou cosmétiques.

Les agents actifs ayant des propriétés cosmétiques peuvent être des hydratants, des émollients, des nettoyants, des relipidants, des agents lipolytiques, des exfoliants, des tenseurs, des agents de pigmentations ou dépigmentation, des parfums et des odorisants.

Les agents actifs peuvent encore être des antiseptiques, des désinfectants et des agents cicatrisants.

Une catégorie d'agents actifs particulièrement visée par la présente invention comprend les agents antibuée.

Les agents antibuée préférés sont des tensioactifs ioniques, non ioniques ou amphotères, de préférence non ioniques.

Une grande variété de tensioactifs peut être employée. Ceux-ci peuvent être ioniques (cationiques, anioniques ou amphotères) ou non ioniques, de préférence non ioniques ou anioniques. Cependant, un mélange de tensioactifs appartenant à ces différentes catégories est envisageable. Ces agents tensioactifs sont pour la plupart disponibles dans le commerce.

De préférence, on utilise un agent tensioactif comportant des groupes poly(oxyalkylène).

Comme exemples de tensioactifs non ioniques utilisables dans la présente invention, on peut citer les poly(alkylènoxy)alkyl-éthers, notamment les poly(éthylènoxy)alkyl-éthers, commercialisés par exemple par CRODA sous la dénominations BRIJ®, les poly(alkylènoxy)alkyl-amines, les poly(alkylènoxy)alkyl-amides, les alcools gras polyéthoxylés, polypropoxylés ou polyglycérolés, les alpha-diols gras polyéthoxylés, polypropoxylés ou polyglycérolés, les alkylphénols gras polyéthoxylés, polypropoxylés ou polyglycérolés et les acides gras polyéthoxylés, polypropoxylés ou polyglycérolés, tous ayant une chaîne grasse comportant par exemple 6 à 20, de préférence 8 à 18 atomes de carbone, le nombre de groupements oxyde d'éthylène ou oxyde de propylène pouvant aller notamment de 2 à 100, préférentiellement 2 à 50 et le nombre de groupements glycérol pouvant aller notamment de 2 à 30, les diols acétyléniques éthoxylés, les composés du type copolymère à blocs comportant à la fois des blocs hydrophiles et des blocs hydrophobes (par exemple des blocs polyoxyéthylène et polyoxypropylène, respectivement), les copolymères poly(oxyéthylène)-poly(diméthylsiloxane) et les tensioactifs incorporant un groupe sorbitane.

Des tensioactifs anioniques préférés sont ceux comprenant un groupe acide sulfonique, on peut citer les alkylsulfosuccinates, les alkyléthersulfosuccinates, les alkylamidesulfosuccinates, les alkylsulfosuccinamates, les sels dibasiques d'acide polyoxyéthylène alkyl sulfosuccinique, les sels dibasiques d'acide alkyl sulfosuccinique, les alkylsulfoacétates, les sels d'hémi-esters d'acide sulfosuccinique, les alkylsulfates et aryl sulfates tels que le dodécylbenzène sulfonate de sodium et le dodécylsulfate de sodium, les sulfates d'alcool gras éthoxylés, les alkyléthersulfates, alkylamidoéthersulfates, alkylarylpolyéthersulfates, les alkylsulfonates, alkylphosphates, les alkylétherphosphates, alkylamidesulfonates, alkylarylsulfonates, $\alpha$-oléfine-sulfonates, les éthoxysulfates d'alcools secondaires, les acides éthers carboxyliques polyoxyalkylénés, les sulfates de monoglycéride, les sels de polyoxyéthylène alkyl éther de l'acide sulfurique, les sels d'esters de l'acide sulfurique, les N-acyltaurates tels que les sels de N-acylméthyltaurine, les sels d'acides monosufonique hydroxyalcanes ou les monosulfonates d'alcènes, le radical alkyle ou acyle de tous ces composés comportant de préférence de 12 à 20 atomes de carbone et l'éventuel groupe oxyalkylène de ces composés comportant de préférence de 2 à 50 unités monomères. Ces tensioactifs anioniques, et bien d'autres, utilisables dans la présente demande, sont décrits dans la demande EP 1418211 et le brevet US 5,997,621.

Comme agents tensioactifs cationiques utilisables dans la présente invention, on peut citer les sels d'amines grasses primaires, secondaires ou tertiaires, éventuellement polyoxyalkylénées, les sels d'ammonium quaternaire tels

que les chlorures ou les bromures de tétraalkylammonium, d'alkylamidoalkyltrialkylammonium, de trialkylbenzylammonium, de trialkylhydroxyalkyl-ammonium ou d'alkylpyridinium, les dérivés d'imidazoline ou les oxydes d'amines à caractère cationique.

Selon un mode de réalisation, le tensioactif utilisé comprend un tensioactif fluoré, préférentiellement amphiphile. Dans le cas d'un tensioactif fluoré, on utilise de préférence ceux comportant au moins un groupe fluoroalkyle ou polyfluoroalkyle et mieux ceux comportant au moins un groupe perfluoroalkyle.

A la place d'une solution de tensioactif, il est possible d'utiliser des composés hydrophiles, plus particulièrement des composés sans propriétés tensioactives comportant au moins un groupe hydrophile, de préférence un groupe poly(oxyalkylène).

Le revêtement antibuée de l'invention présente de préférence un angle de contact statique avec l'eau inférieur ou égal à 10°, mieux inférieur ou égal à 5°lorsqu'il est appliqué sur le substrat pour lequel il est destiné..

Les agents préférés pour des applications antibuée sont décrits ci-après.

Les monoéthers d'alkyle de polyéthylène glycols (A) représentent une première catégorie de tensioactifs préférés. Ils sont de préférence non fluorés. Parmi ceux-ci, on préfèrera utiliser ceux de formule :

$$H(OCH_2CH_2)_nOR^1 \qquad (I)$$

dans laquelle $R^1$ est un groupe alkyle, linéaire ou ramifié, éventuellement substitué par un ou plusieurs groupes fonctionnels, et pouvant comporter en outre une ou plusieurs doubles liaisons, et n est un entier de 1 à 25, de préférence de 2 à 20, mieux de 2 à 15 encore mieux de 4 à 15, et idéalement de 8 à 12. n peut notamment prendre les valeurs 2, 4, 5, 10, ou 20. Selon un mode de réalisation particulier, n est supérieur à 6. Selon un autre mode de réalisation particulier, n est inférieur à 20, mieux inférieur à 15.

$R^1$ est de préférence un groupe alkyle linéaire comprenant de préférence 10 à 20 atomes de carbone, mieux un groupe alkyle linéaire saturé. Des exemples non limitatifs de groupes $R^1$ utilisables sont les groupes dodécyle ($C_{12}H_{25}$), cétyle ($C_{16}H_{33}$), stéaryle ($C_{18}H_{37}$) et oléyle ($C_{18}H_{35}$). Selon un mode de réalisation particulier, le groupe $R^1$ compte 12 atomes de carbone ou moins.

Les tensioactifs de formule (I) ont de préférence une masse molaire de 180 à 1500 g/mol, mieux de 300 à 1000 g/mol et encore mieux de 350 à 800 g/mol.

Des composés de formule (I) utilisables dans la présente invention sont commercialisés par Croda sous la marque Brij®, par exemple les Brij® portant les numéros suivants : C10, L4, C20, S10. Parmi eux, le Brij® C10 (HLB = 12-13) est préféré (composé de formule II avec n = 10 et $R^1$ = n- $C_6H_{13}$).

Les tensioactifs possédant un cycle sorbitane (B) représentent une seconde catégorie de tensioactifs préférés. Parmi ceux-ci, on préfèrera utiliser ceux dont le cycle sorbitane possède n de ses quatre groupes hydroxyle fonctionnalisés avec des groupes polyoxyalkylène à terminaisons OH identiques ou différents (de préférence des groupes polyoxyéthylène), et p de ses quatre groupes hydroxyle fonctionnalisés avec des groupes $R^1$ identiques ou différents de formule : $-(R_dO)_z-(Y)_{n2}-R'$

dans laquelle $R_d$ est un groupe alkylène linéaire ou ramifié, z est un entier ≥ 1, Y est un groupe divalent, n2 représente 0 ou 1 (de préférence, n2 = 1) et R' est un groupe hydrocarboné saturé ayant de 12 à 19 atomes de carbone, de préférence de 13 à 19, n et p étant des entiers tels que n = 2 ou 3 et p = 1 ou 2, avec n + p = 4. De préférence, n = 3 et/ou p = 1.

$R_d$ représente de préférence un groupe alkylène en C2-C6 tel que les groupes propylène ou éthylène, idéalement le groupe éthylène.

L'entier z varie de préférence de 1 à 40, mieux de 2 à 20, encore mieux de 2 à 10.

Les groupes polyoxyalkylène à terminaisons OH de ces composés comprennent de préférence de 1 à 40 groupes oxyalkylène, mieux de 2 à 20, encore mieux de 2 à 10.

Le nombre total de groupes oxyalkylène présents dans la structure des tensioactifs (B) varie de préférence de 4 à 40, mieux de 8 à 30, encore mieux de 15 à 25, et est idéalement égal à 20.

R' est un groupe hydrocarboné saturé ayant de préférence de 14 à 18 atomes de carbone, mieux de 15 à 17. R' est de préférence un groupe alkyle linéaire. R' est de préférence un groupe n-$C_{15}H_{31}$ ou n-$C_{17}H_{35}$.

Des exemples non limitatifs de groupes Y sont les groupes alkylène, cycloalkylène, arylène, carbonyle, amido, ou les combinaisons de ces groupes, linéaires ou ramifiés, optionnellement substitués. Y est de préférence un groupe carbonyle.

Le groupe $-(Y)_{n2}-R'$ est de préférence un groupe palmityle ou un groupe stéaryle.

Les tensioactifs (B) sont de préférence non-ioniques et sont de préférence des esters d'acide gras de polyoxyalkylène sorbitanes, c'est-à-dire des sorbitanes polyoxyalkylénés estérifiés une ou deux fois par un acide gras (Y = carbonyle et n2 = 1), de préférence une seule fois. Mieux, les tensioactifs (B) sont des esters d'acide gras de polyoxyéthylène sorbitanes (Y = carbonyle, n2 = 1 et R = $CH_2CH_2$), en d'autre termes des polysorbates avec des longueurs de chaînes spécifiques pour le groupe ester.

Une classe préférée de tensioactifs (B) comprend les composés de formule (II):

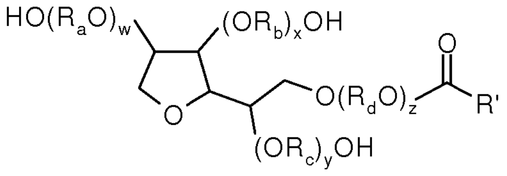

(II)

dans laquelle $R_a$, $R_b$, $R_c$ et $R_d$ représentent indépendamment des groupes alkylène linéaires ou ramifiés, de préférence linéaires, de préférence des groupes alkylène en C2-C6 tels que les groupes propylène ou éthylène, w, x, y et z représentent indépendamment des entiers ≥ 1, de préférence allant de 1 à 40, mieux de 2 à 20, encore mieux de 2 à 10, et R' est tel que défini précédemment.

De préférence, w+x+y+z varie de 4 à 40, mieux de 8 à 30, encore mieux de 15 à 25. Idéalement, w+x+y+z = 20.

Parmi les tensioactifs (B) de formule (II), on préfèrera utiliser les composés polyéthoxylés de formule (II):

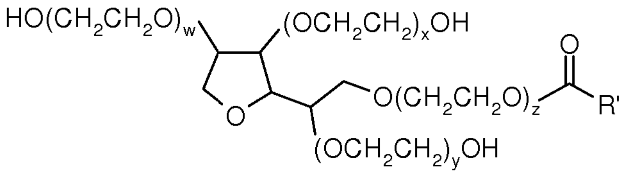

(III)

dans laquelle w, x, y, z et R' sont tels que définis précédemment.

Les tensioactifs (B) peuvent être facilement synthétisés ou sont disponibles dans le commerce. En particulier, les tensioactifs (B) de formule (II) ou (III) sont vendus sous les marques Alkest™, Canarcel™ ou Tween™.

Les tensioactifs (B) préférés sont le Tween™ 40 (HLB = 15,6), également connu sous le nom de monopalmitate de polyoxyéthylène (20) sorbitane (composé de formule X dans laquelle R' = $C_{15}H_{31}$ et w+x+y+z = 20), le Tween™ 60, également connu sous le nom de monostéarate de polyoxyéthylène (20) sorbitane (composé de formule (III) dans laquelle R' = $C_{17}H_{35}$ et w+x+y+z = 20), le Tween™ 20 et le Tween™ 80.

D'autres tensioactifs utilisables sont les copolymères triblocs comprenant deux blocs d'oxyde d'éthylène (EO) et un bloc central d'oxyde de propylène (PPO), dits "poloxamères", commercialisés notamment par BASF sous la dénomination Pluronic®, et notés $(EO)_x$-$(PO)_y$-$(EO)_z$ ou $HO(CH_2CH_2O)_x$-$(CH_2CH(CH_3)O)_y$-$(CH_2CH_2O)_zH$, par exemple les Pluronic® P-123, L-121, P-65, P-64.

D'autres tensioactifs utilisables selon l'invention sont les tensioactifs de nature fluoroalkyle polyéthoxylés, préférentiellement de formule $F(CF_2)_y$-$(CH_2$-$CH_2O)_{x+1}H$ (IV), dans laquelle x et y sont des entiers tels que x varie de 1 à 16 et y est inférieur ou égal à 10.

Parmi ces tensioactifs fluorés, on pourra notamment utiliser le Capstone® FS 3100, le Capstone® FS30, le Capstone® FS 31, le Capstone® FS 34, le Masurf FS 1700, le Masurf FS 1800, le Masurf 2800, le Masurf 2900, le Zonyl® FSO 100 et le Zonyl® FSN 100.

Le Capstone® FS 3100 est un tensioactif comprenant un mélange de composés ayant des longueurs de chaînes polyéthoxylées variables répondant à la formule générale $F(CF_2)_y$-$(CH_2$-$CH_2O)_{x+1}H$ (IV) dont plus de 90% en masse correspond à la fraction y=6 , x étant un entier variant de 1 à 14. Le Capstone® FS3100 contient des teneurs indétectables par HPLC de composé de formule (IV) dans laquelle y est supérieur à 6. Il est biodégradable.

Le Zonyl® FSO 100 (HLB = 9,1), commercialisé par Dupont, lequel est un mélange de composés de formule $F(CF_2)_y$-$(CH_2$-$CH_2O)_{x+1}H$ (IV) dans laquelle y prend les valeurs 6, 8 et 10 dans les proportions massiques respectives de l'ordre de 65%, 30%, 5% et x est un entier variant de 2 à 13.

Selon un mode de réalisation, le tensioactif contient au moins une unité siloxane Si-O et présente en outre une tension superficielle inférieure à 40 mN/m, mieux inférieure à 35 mN/m. Un exemple d'un tel tensioactif est le composé Coatosil 77 commercialisé par Momentive (anciennement Silwet 77, présentant une tension superficielle de 20,5 mN/m), dont la formule est la suivante, n étant égal à 7,5 :

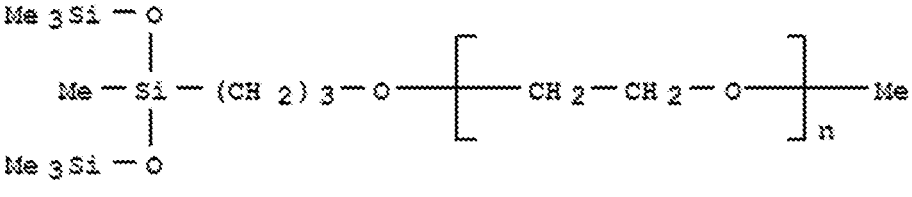

La tension superficielle du tensioactif exprimée en mN/m est obtenue selon la méthode de la plaque de

Wilhelmy : la tension superficielle se mesure pour une solution à 0,1% en masse (solvant : eau). On tire hors du liquide une plaque verticale tout en mesurant la force exercée. On note la valeur de la force d'arrachement de la plaque juste avant que le ménisque se détache. On divise la force ainsi obtenue par la largeur de la plaque, et on obtient la valeur de la tension superficielle. L'angle de contact (0°) entre le liquide et la surface de la plaque doit être garanti par un nettoyage intensif, par exemple par calcination du corps de mesure.

Les tensioactifs envisagés ci-dessus peuvent être utilisés seuls ou en mélange avec un ou plusieurs autres tensioactifs, pour autant que les caractéristiques du mélange considéré restent compatibles avec les propriétés requises selon l'invention. De préférence, les tensioactifs ayant un équilibre hydrophile/lipophile < 5 représentent moins de 10 % de la masse de tensioactifs, mieux moins de 5 % et encore mieux 0 %. De préférence, les tensioactifs ayant un équilibre hydrophile/lipophile > 18 représentent moins de 10 % de la masse de tensioactifs, mieux moins de 5 % et encore mieux 0 %.

Des exemples de tensioactifs préférés sont ceux possédant un équilibre hydrophile/lipophile (HLB) ≥ 5.

Une autre classe particulière d'agents actifs comprend les compositions antimicrobiennes. Par composition antimicrobienne, on entend une composition ayant une activité contre les bactéries et/ou les champignons et/ou les levures et/ou les moisissures. On peut citer les agents biocides, bactériostatiques, bactéricides, anti-levure, antifongiques, fongicides, fongistatiques et/ou répulsifs. Des compositions antimicrobiennes particulières comprennent la combinaison spécifique d'un tensioactif non ionique comprenant un motif hydrophile et un motif hydrophobe, le motif hydrophile contenant des motifs poly(oxylalkylène) de formule $[-R1-O]Z-)]_n$ où R1 représente des groupes alkyène linéaires ou ramifiés, tels que propylène ou éthylène et z est un entier égal ou supérieur à 1, de préférence allant de 1 à 40, le nombre n de ces motifs poly(alkylène) étant égal ou supérieur à 3, et au moins un alcool de faible masse molaire égale à 500g/mol ou moins, le rapport R tensioactif non ionique/alcool étant tel que $2{,}5 \leq R \leq 20$, et de préférence la teneur en poids en alcool, par rapport au poids total de la composition est de 0,01 à 5 %, de préférence 0,01 à 20 %. Les tensioactifs sont les mêmes que ceux décrits ci-dessus, notamment les tensioactifs de formule (I), (II), (III) et (IV).

Parmi les autres agents actifs utilisables dans l'invention on peut citer :

- Certains acides aminés comme le L-tryptophane, la L-lysine, la L-arginine, la L-alanine ;
- Les sucres comme le D-mannitol, le xylitol (le xylitol a une solubilité dans l'eau de 39%) ;
- Les vitamines hydrosolubles comme la vitamine C (acide ascorbique).
- Des composés comme la codéïne, la caféïne, le 5-fluororacil (anticancéreux), l'éthylbutylacétyl-aminopropionate (IR3535).

## Substrats

Les substrats dont les surfaces sont susceptibles d'être traitées avec les particules produites par le procédé selon l'invention sont de nature variée et sont, par exemple, les verres organiques ou minéraux, la peau humaine ou animale, les phanères humains ou animaux, les métaux et alliages, les matières plastiques, les cuirs, etc.

Une classe particulière de substrats sont les verres organiques ou minéraux en particulier des articles d'optique tels que des lentilles ophtalmiques, par exemple des verres de lunettes, dont la surface à traiter peut comporter éventuellement des revêtements fonctionnels, tels que des revêtements antichocs, anti rayures, antireflets, antisalissures, photochromiques, etc.

Un substrat particulier est un article d'optique (lentille optique, par exemple, verre de lunettes, écran, vitrage pour l'industrie automobile ou le bâtiment, miroir) comportant à sa surface un revêtement précurseur de revêtement antibuée. Un tel substrat est plus particulièrement décrit dans la demande internationale WO 2011/080 472.

## Description des figures

Les figures annexées représentent respectivement :

- Fig. 1A, 1B des photographies MEB et optique des microsphères obtenues dans A2 Essai 1 ;
- Fig. 1C, 1D des photographies MEB et optique des microsphères obtenues dans A3 Essai 2 ;
- Fig. 1E photographie MEB et optique A4 essai 3 ;
- Fig. 1F microscopie optique A5 Essai 4 ;
- Fig. 2 est une courbe de distribution granulométrique des microsphères de A5 essai 4 ;
- Fig. 3 observation microscopie optique B Essai1 (IR3535 dans Dynasan 118) ;
- Fig. 4 photographie optique d'une lingette non traitée et d'une lingette imprégnée de microsphères ;
- Figure 5 une photographie optique des microcapsules de l'essai C2 essai 1.

Méthodes de fabrication des particules

De manière générale dans la présente invention les particules sont obtenues selon les méthodes suivantes :

(A) Encapsulation par polycondensation (microcapsules)

(i) - former une émulsion à partir d'une phase huileuse contenant au moins un agent actif ayant une solubilité dans l'eau de 0,1 à 60 % en poids, de préférence de 0,1 % à 30% en poids et d'une phase aqueuse contenant des monomères aptes à être condensés, et
(ii) - effectuer la polycondensation des monomères pour former une membrane définissant une microcapsule, l'agent actif ayant un coefficient de partage mesurable entre la phase huileuse et la phase aqueuse.
le coefficient de partage est défini comme étant le rapport de la concentration de l'agent actif dans la phase huileuse sur la concentration de l'agent actif dans la phase aqueuse. De préférence, le coefficient de partage est supérieur ou égal à 1, mieux supérieur ou égal à 1,5, mieux supérieur ou égal à 2. et

(B) Encapsulation sur des particules déjà formées (microcapsules)

(i) former une particule selon l'invention par la méthode précédente
(ii) former une dispersion des particules dans une phase aqueuse contenant un polymère hydrosoluble,
(iii) rendre insoluble le polymère initialement hydrosoluble pour former des condensats de polymère qui migrent à l'interface des particules, et
(iv) traiter thermiquement ou chimiquement les condensats de polymère pour former une enveloppe sur les particules (formant des microcapsules).

La méthode (A) d'encapsulation par polycondensation, qui concerne la réalisation de microcapsules, est également connue dans son aspect le plus général, notamment pour l'encapsulation au moyen de silicones. Une telle méthode est décrite en particulier dans le brevet EP 2 080 552.

Typiquement dans cette méthode, une émulsion est formée à partir d'une phase huileuse contenant l'agent actif et d'une phase aqueuse contenant des monomères polycondensables (notamment des monomères pour la formation de polysiloxanes). Lors du processus, les monomères migrent à l'interface des gouttelettes (du fait du pH ou de la force ionique) et on initie leur polycondensation par modification de pH ou de la température pour former la membrane des microcapsules emprisonnant la phase huileuse contenant l'agent actif.

Selon l'invention cette méthode est modifiée par le choix de la phase huileuse, notamment une huile fluorée, qui est choisie de façon à ce que l'agent présente un coefficient de partage élevé entre la phase huileuse et la phase aqueuse (et donc que la concentration d'agent dans la phase aqueuse à l'équilibre soit minimale). Comme indiqué précédemment, le coefficient de partage est défini comme étant le rapport de la concentration de l'agent dans la phase huileuse sur la concentration de l'agent dans la phase aqueuse. De préférence, le coefficient de partage est supérieur ou égal à 1, mieux supérieur ou égal à 1, 5, mieux supérieur ou égal à 2 (mesuré à 25°C).

A titre d'exemple, pour la réalisation de capsules antibuée, l'agent actif peut être le Zonyl® FSO100 et l'huile fluorée, l'huile HFE 7300.

Outre le fait que les microcapsules obtenues contiennent en leur coeur l'agent actif, elles ont l'avantage de permettre une libération facile de l'agent actif et d'avoir une résistance élevée à la température, de l'ordre de 120°C.

La méthode (B) d'encapsulation est connue dans son aspect général et décrite notamment dans le brevet EP 1 533 415. Selon un mode de réalisation, la méthode (B) est mise en oeuvre et l'enveloppe des particules est un polymère mélamine/formaldéhyde.

Cependant, la méthode (B) ne peut pas être appliquée directement à la formation de microcapsules contenant un agent actif partiellement hydrosoluble. Ainsi, selon l'invention, cette méthode est mise en oeuvre en partant de particules insolubles dans l'eau contenant un agent actif tel que défini.

Les particules sont dispersées dans une phase aqueuse contenant un polymère initialement hydrosoluble. On rend ensuite le polymère insoluble, par exemple par modification du pH ou ajout de sels. Il se forme alors des petites particules solides de polymère (condensats) qui migrent à l'interface des gouttelettes et forment ensuite sous l'action d'un traitement thermique ou chimique une membrane sur les particules.

Cette technologie est particulièrement adaptée pour conférer aux particules de l'invention une enveloppe capable de se greffer par une liaison covalente au support.

Le procédé B est décrit plus précisément, mais non limitativement ci-après.

Le procédé B comporte l'étape suivante, ou de préférence consiste à encapsuler à nouveau des microcapsules obtenues à l'aide du procédé A dans une membrane.

Les nouvelles microcapsules peuvent ensuite être fixées sur fibres (par l'intermédiaire d'un liant par exemple),

être libérées par frottements, et présenter une résistance aux lavages.

Les étapes principales du procédé sont les suivantes :

- Préparation d'une solution aqueuse contenant un polymère (ou pré-polymère) soluble dans l'eau. Le polymère peut être choisi parmi les produits dont la solubilité peut être contrôlée par le pH. Les produits peuvent être choisis parmi les résines aminoplastes, telles que les résines urée-formol et les résines mélamine-formol, ou les copolymères d'acide acrylique et méthacrylates de méthyle (comme la gamme Eudragit® poly(methacylic acid-co-methyl methacrylate) 1:2) tels que l'Eudragit® L100 ;
- Mélange de la solution de polymère avec les microcapsules à enrober ;
- Modification de la solubilité du polymère par abaissement du pH.

Pour les résines aminoplastes, le pH peut être ajusté dans la gamme 3,5 à 4,5, préférentiellement dans la gamme 3,7 à 4,2, typiquement 4, par ajout d'acide formique par exemple. La désolvatation du polymère provoque la formation de condensats.

Pour les copolymères d'acide acrylique et méthacrylates de méthyle Eudragit®, le pH peut être abaissé à 5 ou moins par ajout d'un acide fort comme l'APTS (acide paratoluène sulfonique) ou par un acide faible comme l'acide acétique. Typiquement, l'Eudragit® L100 est soluble pour des pH au-dessus de 5,5 et insoluble en dessous ;

- Migration des condensats à l'interface eau/microcapsules permettant la formation d'une membrane de polymère autour des microcapsules pour former de nouvelles microcapsules.
- Durcissement de la membrane par augmentation de la température au-dessus de la température de réticulation pour les résines aminoplastes, ou par augmentation de la température au-dessus de la température permettant la formation d'un film de polymère autour des microcapsules.
- Dans le cas des membranes aminoplastes, les microcapsules peuvent être appliquées sur le textile par l'intermédiaire des procédés textiles connus et fixés chimiquement (par liaisons covalentes ou par l'intermédiaire d'un liant). Dans le cas des membranes Eudragit® L100, les microcapsules peuvent être appliquées sur le textile à partir des procédés connus de l'industrie textile, généralement à un pH inférieur ou égal à 5.

L'invention concerne également l'utilisation d'un support tel que présenté ci-dessus pour conférer ou réactiver une fonction sur une surface d'un substrat, dans laquelle le support est appliqué sur le substrat sous l'effet d'une contrainte externe pour libérer le (ou les) agent(s) actif (s).

La contrainte externe peut être, sans limitation, une contrainte mécanique, thermique ou chimique.

Dans un mode de réalisation, le substrat est une lentille ophtalmique telle qu'un verre de lunette.

Dans un autre mode de réalisation, le substrat est la peau humaine ou animale. Dans ce cas, l'utilisation du support peut notamment être une utilisation thérapeutique (par exemple dermatologique), cosmétique, cosmétique et non thérapeutique ou non thérapeutique, selon la nature du ou des agents actifs.

L'invention est illustrée, de façon non limitative, par les exemples suivants.

<u>EXEMPLES</u>

**Matériel et méthodes**

Les particules obtenues ont été caractérisées visuellement par microscopie optique et électronique

- microscope optique
- microscope à balayage électronique

La distribution de taille des particules non solubles dans l'eau a été mesurée avec un granulomètre laser Malvern mastersizer 2000.

L'atomisation est réalisée sur une tour SD1 équipée d'une buse à mélange interne co-courant, fournie par la société TechniProcess.

Le pilote D1 est une tour de séchage par atomisation qui a été conçue pour avoir une capacité évaporatoire comprise entre 1 et 3kg/h et un débit d'air de séchage avoisinant les 100 kg/h .

**A. Description des essais d'encapsulation par séparation de phase réalisés (microsphères)**

**1. Mode opératoire pour la réalisation des essais**

Préparation de la solution à atomiser contenant : la matrice, l'actif à encapsuler et les additifs si nécessaire en milieu aqueux.

Atomisation de la solution sur une tour de séchage et récupération des particules sous forme de poudre.

**2. Essai 1 : Ethyl butylacetylaminopropionate / gomme d'acacia :**

Formulation à pH 2 et à 50°C :
500g de solution dont la formulation est propice à l'encapsulation par séparation de phase pour placer l'actif au centre des capsules :

| Désignation | Taux |
|---|---|
| Gomme d'acacia | 24% |
| Ethyl butylacétylaminopropionate | 8% |
| Acide para toluène sulfonique (APTS) | 4% |
| Eau | 64% |

Teneur en actif estimée dans la poudre 25 % [8/(24+8)]
NB : L'ajout d'APTS permet d'augmenter la solubilité de l'éthylbutylacetylaminopropionate.

Paramètres de pulvérisation :

Température d'entrée 180°C
Température de sortie 85-90°C
Pression buse 2 bars

**3. Essai 2 : Xylitol / gomme d'acacia :**

Formulation à température ambiante et pH neutre :
500g de solution dont la formulation est propice à l'encapsulation par séparation de phase pour placer l'actif au centre des capsules
Le xylitol a une solubilité dans l'eau à 25°C de 30% massique.

| Désignation | Taux |
|---|---|
| Gomme d'acacia | 21% |
| Xvlitol | 9% |
| Eau | 70% |

Teneur en actif estimée dans la poudre 30 %

Paramètres d'atomisation :

Température d'entrée 180°C
Température de sortie 85,5°C
Pression de la buse 3 bars

Poudre grisâtre (acacia pulvérisé), aspect habituel de l'acacia pulvérisé, odeur acacia, pas de reprise en eau

**4. Essai 3 : NaCl/gomme d'acacia**

Formulation à température ambiante et pH neutre :
500g de solution dont la formulation est propice à l'encapsulation par séparation de phase pour placer l'actif au centre des capsules

| **Désignation** | **Taux** |
|---|---|
| Gomme d'acacia | 21% |
| NaCl | 9% |
| Eau | 70% |

Teneur en actif estimée dans la poudre 30 %

Le NaCl a une solubilté dans l'eau de 26% en poids à 25°C.

Conditions d'atomisation :

Température d'entrée 180°C
Température de sortie 80°C
Pression de la buse 3 bars

La structure cristalline du sel étant cubique, le cliché 3E permet d'observer que les cristaux de sel sont situés à l'intérieur de la capsule (emplacement souhaité).

**5. Essai 4 : Zonyl FSO100 / Ethylcellulose SD_1023.066**

Formulation à froid et pH neutre

300g de solution propice à l'encapsulation par séparation de phase pour placer l'actif au centre des capsules.

Ethylcellulose disponible commercialement en suspension dans l'eau à 30% (Aquacoat ECD)

| **Désignation** | **Taux** |
|---|---|
| Aquacoat ECD en suspension (30% d'extrait sec) | 50% (15% sec) |
| Zonyl | 9% |
| Triacetine | 3% |
| Eau | Qsp 100% |

Teneur en actif estimée dans la poudre 33 %

Conditions d'atomisation :

Température d'entrée 180°C
Température de sortie 80°C
Pression de la buse 3 bars

**B. Description de l'encapsulation de l'IR3535 par le procédé Creaspher adapté (microsphères)** (cet exemple n'est pas selon l'invention)

**1. Mode opératoire**

Le slurry de microsphères est préparé de la façon suivante :

1. préparation de la phase grasse contenant la cire et le principe actif à encapsuler, à 10°C au-dessus de la température de fusion de la cre utilisée
2. préparation de la phase aqueuse 1 contenant un tensio-actif, un colloïde protecteur comme la gomme d'acacia, et chauffage à la même température que la phase grasse
3. Ajout de la phase grasse fondue dans la, phase aqueuse 1 et réalisation d'une émulsion pendant 10 minutes sous agitation (IKA pale défloculeuse, 1200 tr/min)
4. Réalisation de la phase aqueuse 2 contenant un tensioactif et un colloïde protecteur tel que la gomme d'acacia, refroidissement de la solution à 4°C
5. Transfert au goutte à goutte de la première émulsion dans la seconde phase aqueuse sous agitation (pale de malaxage, 1000 tr/min)

Les particules sont récupérées en suspension dans l'eau.

**Essai 1 : Ethyl butylacetylaminopropionate (IR3535) dans Dynasan 118 (figure 3)**

| ***Phase*** | ***Produits / Etapes*** | ***M (g)*** |
|---|---|---|
| Phase grasse | Huile: dynasan 118<br>Cire cosmétique. Température de fusion 70-74°C | 84,00 |
| | Actif : Ethyl butylacetylaminopropionate | 56,00 |
| Phase aqueuse 1 | Eau | 129,2 |
| | Brij721P | 0,72 |
| | fibregum bio | 10,08 |
| Phase aqueuse 2 | Eau | 103,56 |
| | Ethyl butylacetylaminopropionate | 7,2 |
| | Fibregum bio | 8,64 |
| | Brij721P | 0,60 |

L'IR3535 a une solubilité dans l'eau à 20°C d'environ 7% massique.
Au-delà, on observe deux phases : une phase aqueuse contenant 7% d'IR3535 et une phase liquide dIR3535 seule.
Les particules obtenues ont les caractéristiques suivantes :

| | |
|---|---|
| **Granulométrie** | 12,6 μm |
| **Concentration en actif encapsulé (estimé)** | 12,6 % dans le slurry<br>37 % dans le slurry sec<br>40 % dans la capsule |
| **Extrait sec théorique** | 41,8 % |

**Application** : **Dépose des capsules par foulardage sur textile CEMOI**

Le bain d'imprégnation des lingettes est composé de microsphères d'ethyl butylacetylaminopropionate / Dynasan 118 préparées dans l'exemple précédent et diluées dans l'eau ; la concentration du bain en microsphères étant

de 24.5%. Les paramètres de foulardage (concentration du bain et réglages de l'appareil) sont définis afin d'optimiser la quantité déposée en fonction du taux d'emport.

$$\underline{\textbf{Calcul du taux d'emport (en \%)}} : \frac{(\text{masse}_{\text{textile+dépôt}} - \text{masse}_{\text{textile}})}{\text{masse}_{\text{textile}}} \times 100$$

Après avoir défini le taux d'emport du textile, la concentration de microsphères dans le bain est adaptée de manière à déposer une quantité déterminée de microsphères sur le textile. Les paramètres de foulardage ainsi choisis permettent un dépôt de microsphères de 15 μm, tout en conservant leur intégrité (forme sphérique) équivalent à environ 130 mg d'actif par lingette

Le séchage des lingettes est réalisé à plat, et à température ambiante afin de limiter l'évaporation de l'actif liée au séchage à l'étuve.

**C. Description de l'encapsulation par polycondensation (microcapsules)**

**1. Mode opératoire**

1. Réalisation d'une phase aqueuse acide contenant un tensioactif cationique et des stabilisants
2. Préparation de la phase grasse contenant l'actif solubilisé.
Afin d'eviter que le principe actif amphiphile ne migre dans la phase aqueuse lors de la réalisation de l'émulsion celui-ci est solubilisé dans un solvant tel que le coefficient de partage du composé actif dans la phase aqueuse soit le plus faible possible.
3. Réalisation d'une émulsion huile dans eau
4. Ajout des monomères silanes dans l'émulsion. Palier de deux heures pendant lesquelles à lieu l'hydrolyse acide des silanes en silanols et la migration des monomères à l'interface de l'emulsion.
5. Augmentation du pH par ajout d'une base et formation de la membrane par réaction de polycondensation des silanols situés a l'interface.
6. Neutralisation du milieu

On récupère les particules sous forme de slurry.

**2. Essai 1** : **Zonyl FSO100 dans silicone**

| ***Phases*** | ***Produits*** | ***M (g)*** |
|---|---|---|
| Phase aqueuse acide | Eau | 53,02 |
| | Tylose H15YG4 hydroxyethylcellulose | 0,57 |
| | CMC 7LC carboxymethylcellulose | 0,12 |
| | Tensioactif cationique : Crodacel QM | 1,13 |
| | Volpo L3 Spécial | 0,38 |
| | Acide acétique | 3,79 |
| | Acide formique | 1,13 |
| Phase grasse | Actif : Zonyl FSO 100 | 3,89 |
| | Solvant : HFE 7300 | 34,91 |
| Membrane silicone | Dynasylan A | 7,91 |
| | Dynasylan MTES | 7,91 |

(suite)

| *Phases* | *Produits* | *M (g)* |
|---|---|---|
| Milieu basique pour polycondensation | NaOH | 8,99 (qsp pH 5.5) |
| | NaOH | quantité suffisante pour pH 7.5 |
| Antimicrobien | Symdiol 68T | 1,25 |

Rôle du Tvlose et CMC : modificateurs de rhéologie

Les particules obtenues ont les caractéristiques suivantes :

| | |
|---|---|
| **Granulométrie :** | *8,38μm* |
| **Taux d'actif estimé :** | *33,1%* |
| **Extrait sec (65°C) :** | *18,3%* |
| **pH :** | *7,75* |

**D. Description de l'encapsulation par mélamine autour de capsules déjà formées (microcapsules)**

1. Mise en solution du polymère hydrosoluble (mélamine) avec un tensioactif à 35°C
2. Mélange de la solution de polymère avec les capsules lavées et séchées à enrober
3. Ajout de l'acide formique : le polymère réagit avec le formol et se condense à la surface des capsules pour commencer à former la membrane
4. Augmentation de la température à 80°C (1 °C/min pendant 45 min) : la membrane se rigidifie
5. Ajout de mélamine et acide formique (en continu pendant 90 min) réticulation de la paroi et neutralisation du formol en excès. Pendant cette étape le pH doit être maintenu en dessous de 4.5.
6. Refroidissement et augmentation du pH avec une solution de diéthanolamine (l'ajout de DEA permet de consommer le formol résiduel.
7. Les capsules sont récupérées sous forme de slurry.

**Revendications**

**1.** Procédé de fabrication d'une particule se présentant sous la forme d'une microcapsule comprenant une enveloppe solide renfermant une phase fluide ou solide dans laquelle est présent au moins un agent actif, **caractérisé en ce que** le procédé comprend :

(A)

(i) - former une émulsion partir d'une phase huileuse contenant une huile fluorée, au moins un agent actif ayant une solubilité dans l'eau à 20°C de 0,1 à 60 % en poids, de préférence de 0,1 % à 30% en poids et d'une phase aqueuse contenant des monomères aptes à être condensés, et
(ii) - effectuer la polycondensation des monomères pour former une membrane définissant une microcapsule, la phase huileuse comprenant une huile fluorée étant choisie de manière à ce que l'agent actif présente un coefficient de partage entre cette phase huileuse et la phase aqueuse supérieur ou égal à 1, le coefficient de partage étant défini comme le rapport de la concentration de l'agent actif dans la phase huileuse sur la concentration de l'agent actif dans la phase aqueuse.

**2.** Procédé selon la revendication 1, **caractérisé en ce que** ledit agent actif a une solubilité dans l'eau à 20°C de 0,1 à 30% en poids.

**3.** Procédé selon la revendication 1 ou 2, **caractérisé en ce qu'**il comprend en outre :
(B)

(i) former une dispersion des particules dans une phase aqueuse contenant un polymère hydrosoluble,
(ii) rendre insoluble le polymère initialement hydrosoluble pour former des condensats de polymère qui migrent à l'interface des particules, et
(iii) traiter thermiquement ou chimiquement les condensats de polymère pour former une enveloppe sur les particules.

4. Procédé selon la revendication 3, **caractérisé en ce que** l'enveloppe sur les particules est un polymère mélamine/formaldéhyde.

5. Procédé selon l'une quelconque des revendications précédentes, **caractérisé en ce que** les monomères sont des monomères pour la formation de polysiloxanes.

6. Procédé selon l'une quelconque des revendications précédentes, **caractérisé en ce que** la polycondensation des monomères est effectuée par modification de pH ou de la température.

## Patentansprüche

1. Verfahren zur Herstellung eines Teilchens, das in Form einer Mikrokapsel mit einer festen Hülle, die eine flüssige oder feste Phase, in welcher mindestens ein Wirkstoff vorliegt, umgibt, vorliegt, **dadurch gekennzeichnet, dass** das Verfahren Folgendes umfasst:

(A)

(i) - Bilden einer Emulsion aus einer öligen Phase, die ein fluoriertes Öl und mindestens einen Wirkstoff mit einer Wasserlöslichkeit bei 20 °C von 0,1 bis 60 Gew.-%, vorzugsweise 0,1 bis 30 Gew.-%, umfasst, und einer wässrigen Phase, die kondensationsfähige Monomere umfasst, und
(ii) - Durchführen der Polykondensation der Monomere zur Bildung einer eine Mikrokapsel definierenden Membran, wobei die ölige Phase, die ein fluoriertes Öl umfasst, so gewählt wird, dass der Wirkstoff einen Verteilungskoeffizienten zwischen dieser öligen Phase und der wässrigen Phase größer oder gleich 1 aufweist, wobei der Verteilungskoeffizient definiert ist als das Verhältnis der Konzentration des Wirkstoffs in der öligen Phase zur Konzentration des Wirkstoffs in der wässrigen Phase.

2. Verfahren nach Anspruch 1, **dadurch gekennzeichnet, dass** der Wirkstoff eine Wasserlöslichkeit bei 20 °C von 0,1 bis 30 Gew.-% aufweist.

3. Verfahren nach Anspruch 1 oder 2, **dadurch gekennzeichnet, dass** es außerdem Folgendes umfasst:
(B)

(i) Bilden einer Dispersion der Teilchen in einer wässrigen Phase, die ein wasserlösliches Polymer enthält,
(ii) Unlöslichmachen des anfänglich wasserlöslichen Polymers zur Bildung von Polymerkondensaten, die zur Grenzfläche der Teilchen wandern, und
(iii) thermisches oder chemisches Behandeln der Polymerkondensate zur Bildung einer Hülle auf den Teilchen.

4. Verfahren nach Anspruch 3, **dadurch gekennzeichnet, dass** es sich bei der Hülle auf den Teilchen um ein Melamin/Formaldehyd-Polymer handelt.

5. Verfahren nach einem der vorhergehenden Ansprüche, **dadurch gekennzeichnet, dass** es sich bei den Monomeren um Monomere zur Bildung von Polysiloxanen handelt.

6. Verfahren nach einem der vorhergehenden Ansprüche, **dadurch gekennzeichnet, dass** die Polykondensation der Monomere durch Modifizierung des pH-Werts oder der Temperatur durchgeführt wird.

## Claims

1. A process for the manufacture of a particle provided in the form of a microcapsule comprising a solid casing including a fluid or solid phase in which at least one active agent is present, **characterized in that** the process comprises:

(A)

(i) - forming an emulsion from an oily phase containing a fluorinated oil, at least one active agent having a solubility in water at 20°C of 0.1% to 60% by weight, preferably of 0.1% to 30% by weight, and from an aqueous phase containing monomers capable of being condensed; and
(ii) - carrying out the polycondensation of the monomers in order to form a membrane defining a microcapsule, the oily phase comprising a fluorinated oil being chosen so that the active agent exhibits a partition coefficient between this oily phase and the aqueous phase higher than or equal to 1, the partition coefficient being defined as the ratio of the concentration of the active agent in the oily phase to the concentration of the active agent in the aqueous phase.

2. Process according to claim 1, **characterized in that** said active agent has a solubility in water at 20°C of 0.1% to 30% by weight.

3. Process according to claim 1 or 2, **characterized in that** it further comprises:
(B)

(i) - forming a dispersion of the particles in an aqueous phase containing a water-soluble polymer;
(ii) - rendering the initially water-soluble polymer insoluble in order to form polymer condensates which migrate to the interface of the particles; and
(iii) - treating the polymer condensates thermally or chemically in order to form a casing on the particles.

4. Process according to claim 3, **characterized in that** the casing on the particles is a melamine-formaldehyde polymer.

5. Process according to any one of the preceding claims, **characterized in that** the monomers are monomers for forming polysiloxanes.

6. Process according to any one of the preceding claims, **characterized in that** the polycondensation of the monomers is carried out by modifying the pH or temperature.

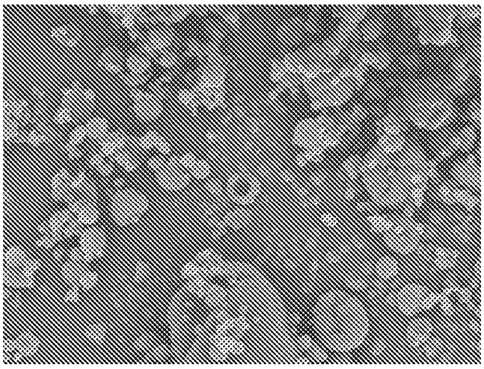
FIGURE 1A

*Photographie MEB des microcapsules acacia/Ethyl butylacetylaminopropionate (75/25)*

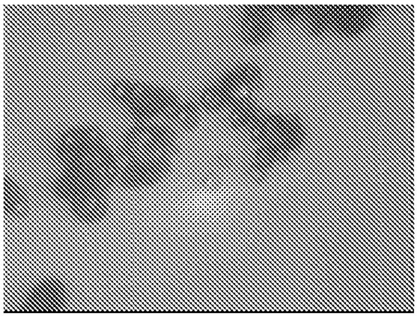
FIGURE 1B

*Photographie optique à 400x et 1000x des microcapsules acacia/Ethyl butylacetylaminopropionate*

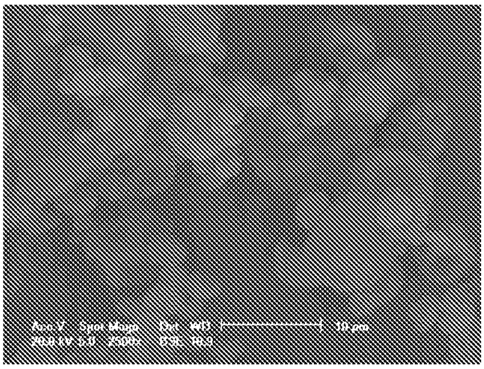
FIGURE 1C

Photographie MEB des microcapsules acacia /xylitol

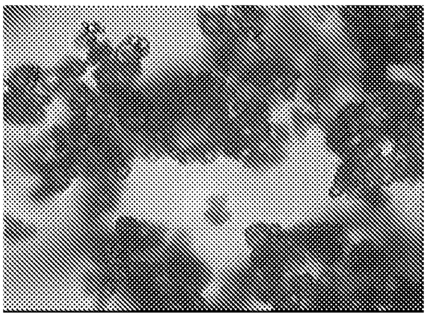
FIGURE 1D

***Photographie optique à 400x***

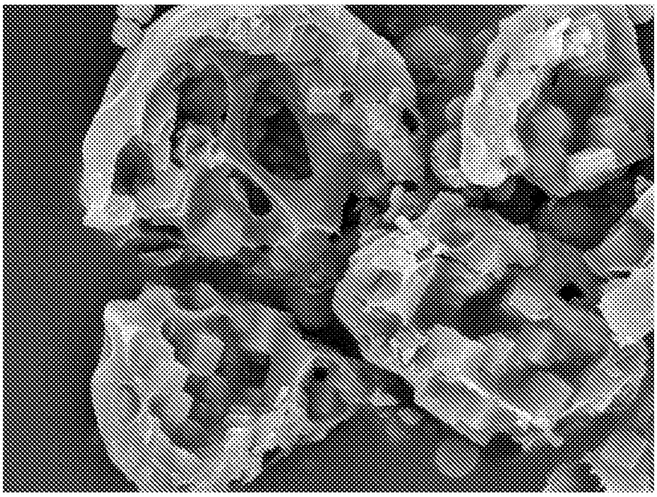

FIGURE 1E

MEB, cliché x2000

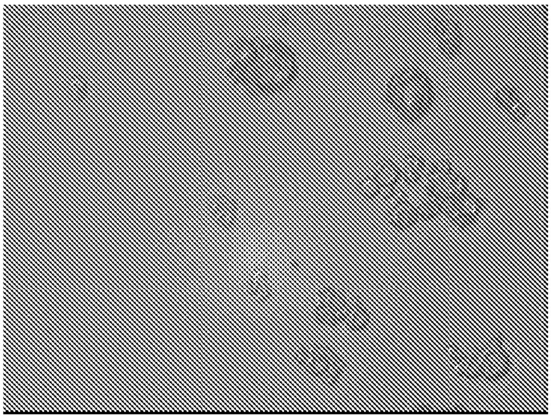

FIGURE 1F

Observation microscopie optique x 400

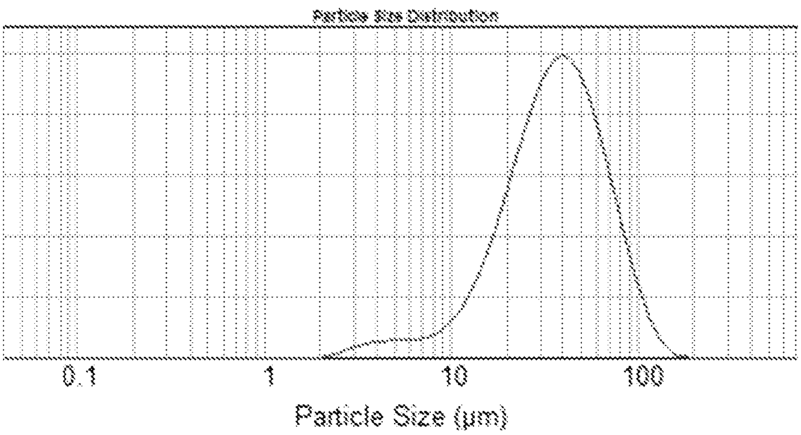


FIGURE 2

Distribution granulométrique des particules obtenues d(0.5)=40µm

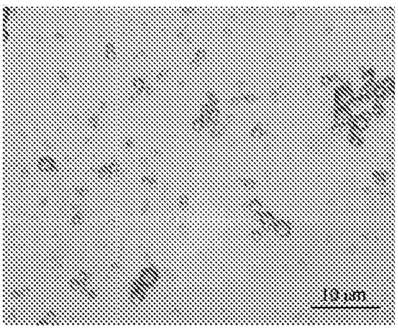


FIGURE 3

**Observation au microscope optique x400**

| Lingette non traitée | Lingettes avec capsules déposées par foulardage |
| --- | --- |
| 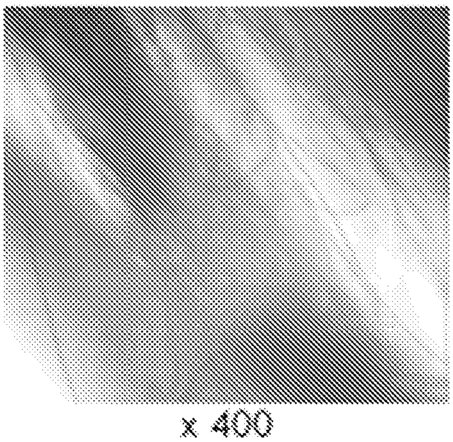<br>x 400 | 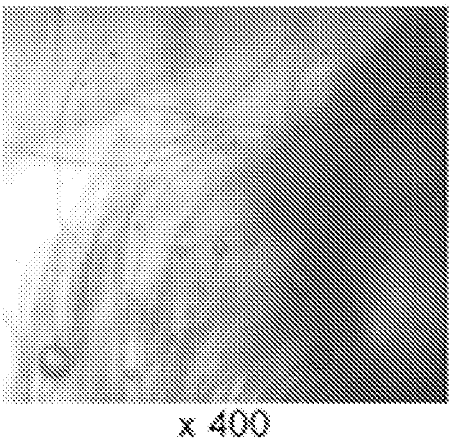<br>x 400 |

FIGURE 4

Microscopie optique réalisée sur les lingettes avant et après traitement

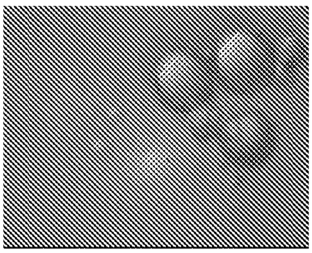

**Microscope optique (x1000)**

FIGURE 5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- FR 2868684
- JP 2009195648 A
- WO 2013013929 A
- JP 02300387 A
- JP 2005296920 A
- EP 2080552 A
- WO 2012038666 A
- FR 2995222
- EP 1533415 A
- EP 0407257 A
- US 2002055560 A
- GB 1242689 A
- FR 2995222 A
- JP H02300387 A
- JP 2004360157 A
- US 6951836 B
- US 2011200654 A
- EP 1418211 A
- US 5997621 A
- WO 2011080472 A